# EUROPÄISCHE PATENTSCHRIFT

(11) **EP 2 160 986 B1**
(45) Veröffentlichungstag und Bekanntmachung des Hinweises auf die Patenterteilung: **18.05.2011**
(21) Anmeldenummer: 08163759.7
(22) Anmeldetag: 05.09.2008
(51) Int. Cl.: A61B 17/32, A61B 19/00

(54) **Vorrichtung zur Befestigung einer Markervorrichtung an einem Knochen**
Device for attaching a marker device to a bone
Dispositif de fixation d'un dispositif de marqueur sur un os

(43) Veröffentlichungstag der Anmeldung: 10.03.2010
(73) Patentinhaber: BrainLAB AG, 85622 Feldkirchen (DE)
(72) Erfinder: Warkentine, Blaine, draper, 84020 (US); Plassky, Norman, 99099, Erfurt (DE); Millahn, Manuel, 80799, München (DE)
(74) Vertreter: Schwabe - Sandmair - Marx

(56) Entgegenhaltungen:
- EP-A- 1 561 431
- EP-A- 1 588 672
- WO-A-00/48508
- DE-U1- 20 103 416
- US-A1- 2004 068 260
- US-B1- 6 203 543

## Beschreibung

Die vorliegende Erfindung betrifft eine Vorrichtung zur Befestigung einer Markervorrichtung an einem Knochen, aufweisend ein Fixierelement und eine Halterung für die Markervorrichtung am Fixierelement.

Bei bildgestützten Operationen ist es notwendig, Objekte wie Knochen oder medizinische Instrumente mit Markervorrichtungen zu versehen. Eine Markervorrichtung besteht beispielsweise aus einer oder mehreren reflektierenden Kugeln, die von Sensoren erfasst werden. Aus der Position der Kugeln zueinander kann die Lage der Markervorrichtung und damit die Lage des Objekts bestimmt werden.

Das Dokument WO 00/48508, von dem der Oberbegriff des Anspruchs 1 abgeleitet ist, offenbart eine Vorrichtung zur Befestigung einer Markervorrichtung an einem Knochen. Dazu wird eine Hülse, die eine Halterung für die Markervorrichtung trägt, mittels einer Schraube am Knochen angebracht. Die Hülse ist um die Schraube drehbar und über eine Feder am Knochen abgestützt.

Das Dokument EP 1 588 672 A1 offenbart eine Vorrichtung zur Befestigung einer Markervorrichtung an einem medizinischen Instrument. Dazu wird die Vorrichtung in eine Ausnehmung des medizinischen Instruments gesteckt und das eingesteckte Ende mittels eines Federmechanismus gespreizt.

Das Dokument EP 1 561 431 A1 offenbart eine Markervorrichtung, die an einem Schneidblock befestigt werden kann. Dabei werden zwei Zungen der Vorrichtung, die in den Schneidschlitz des Schneidblocks eingeführt werden, mittels einer Feder auseinandergedrückt.

Eine weitere am Markt erhältliche Vorrichtung zur Befestigung einer Markervorrichtung an einem Knochen besteht aus einer Schraube, einer Klemmvorrichtung, einer Stellschraube und einer Hülse, die eine Halterung für die Markervorrichtung aufweist. Bei der Anbringung wird zunächst die Schraube in den Knochen geschraubt und anschließend die Hülse mit der Stellschraube sowie die Klemmvorrichtung auf die Schraube aufgeschoben. Dann wird die Klemmvorrichtung gegenüber der Schraube fixiert und die Hülse in der gewünschten Ausrichtung mittels der Stellschraube gegen den Knochen gedrückt und somit fixiert.

Einer der Nachteile dieser Befestigungsvorrichtung besteht darin, dass deren Anbringung am Knochen eine Vielzahl von Arbeitsschritten am Patienten erfordert und somit die Zeit, die der Patient im Operationssaal verbringen muss, verlängert wird.

Es ist die Aufgabe der vorliegenden Erfindung, eine Vorrichtung zur Befestigung einer Markervorrichtung an einem Knochen bereitzustellen, die die vorstehend genannten Nachteile nicht aufweist.

Gelöst wird diese Aufgabe durch die Vorrichtung gemäß Patentanspruch 1. Vorteilhafte Ausgestaltungsformen sind den abhängigen Patentansprüchen zu entnehmen.

Eine Vorrichtung zur Befestigung einer Markervorrichtung an einem Knochen weist ein Fixierelement mit einer Halterung für die Markervorrichtung sowie eine zum Kontakt mit einem Anschlag ausgebildete Feder zur Ausübung einer Kraft zwischen dem Anschlag und dem Fixierelement in Richtung des Knochens auf Die Vorrichtung ist dazu ausgebildet, mittels einer Schraube am Knochen fixiert zu werden. Dazu weist die Vorrichtung eine Durchführung auf, durch die eine Schraube hindurchführbar ist und im bestimmungsgemäßen Gebrauch der Vorrichtung hindurchgeführt ist. Diese Durchführung verläuft vorzugsweise durch das Fixierelement und die Feder. Die Durchführung ist bevorzugt mittig im Fixierelement angeordnet, um die von der Schraube auf die Vorrichtung ausgeübten Momente zu minimieren. Die Kombination aus der erfindungsgemäßen Vorrichtung und der Schraube bildet ein Befestigungssystem, wobei sich die Schraube durch das Fixierelement und die Feder erstreckt.

Das erfindungsgemäße Befestigungssystem hat den Vorteil, dass die Befestigungsvorrichtung bereits vor dem Einschrauben der Schraube, also vor der Einbeziehung des Patienten, auf die Schraube aufgeschoben werden kann. Außerdem entfallen Kräfte, die bei der Betätigung der Stellschraube auf die Befestigungsvorrichtung und damit den Patienten wirken. Darüber hinaus drückt die Feder aufgrund ihrer Vorspannung das Fixierelement automatisch gegen den Knochen, ohne dass eine Stellschraube betätigt werden muss.

Dazu ist das Fixierelement bevorzugt gegenüber der Schraube in Richtung der Schraubenachse verschiebbar angeordnet. Die Feder ist so angeordnet, dass sie das Fixierelement relativ zur Schraube gegen den Knochen drückt. Dies fixiert das Fixierelement gegenüber dem Knochen. Das Fixierelement hält die Vorspannung auch bei sich setzender Schranke oder Hülse und Knochen aufrecht und sorgt damit dafür, dass die Rotationsstabilität durch ständiges Eingreifen der Verzahnung im Knochen gewährleistet ist. Weiterhin hat die erfindungsgemäße Vorrichtung den Vorteil, dass das Fixierelement automatisch tiefer in den Knochen hineingedrückt wird, wenn der Knochen im Laufe der Zeit weicher wird, sodass auch dann ein guter Halt der Befestigungsvorrichtung gewährleistet ist.

Weiterhin bevorzugt ist das Fixierelement gegenüber der Schraube um die Schraubenachse verdrehbar angeordnet. Somit kann das Fixierelement unabhängig von der Drehstellung der Schraube gegenüber dem Knochen in der gewünschten Ausrichtung fixiert werden.

Von besonderer Wichtigkeit ist, dass die Befestigungsvorrichtung derart im Knochen verankert wird, dass ein auf die Vorrichtung wirkendes Moment die Lage der Vorrichtung zum Knochen nicht verändert. Dazu wird die Schraube nach dem Stand der Technik bikortikal in den Knochen eingeschraubt, Die bikortikale Verschraubung ist auch mit der vorliegenden Befestigungsvorrichtung möglich, jedoch nicht zwingend erforderlich.

In einer Ausgestaltungsform der Erfindung weist die dem Knochen zugewandte Seite des Fixierelements eine Stützplatte auf. Durch die Stützplatte liegt das Fixierelement großflächig auf dem Knochen auf, wodurch eine unikortikale Verschraubung ausreichend sein kann, um auf die Vorrichtung einwirkende Momente aufzunehmen. Die Stützplatte ist beispielsweise einstückig mit dem Fixierelement ausgebildet oder als separates Bauteil, das fest, beispielsweise durch Verschraubung, mit dem Fixierelement verbunden ist.

Bevorzugt weist die Vorrichtung mindestens einen außermittig angeordneten Spike an der dem Knochen zugewandten Seite des Fixierelements auf. Übt die Feder eine Kraft auf das Fixierelement aus, so wird der Spike in den Knochen hineingedrückt und verhindert so eine Verdrehung des Fixierelements gegenüber dem Knochen.

Der Anschlag ist beispielsweise Teil der Schraube, zum Beispiel der Kopf einer Kopfschraube oder ein Vorsprung, also eine radiale Verdickung einer Schanz-Schraube. Alternativ weist die Befestigungsvorrichtung ein Anschlagelement mit einem Anschlag für die Feder auf, wobei die Feder so angeordnet ist, dass sie eine Kraft zwischen dem Anschlagelement und dem Fixierelement ausübt, die die Befestigungsvorrichtung spreizt. Das Anschlagelement ist in Richtung der Schraubenachse gegenüber dem Fixierelement verschiebbar. Das Anschlagelement wird von der Schraube abgestützt, beispielsweise vom Kopf einer Kopfschraube oder einem Vorsprung, also einer radialen Verdickung, einer Schanz-Schraube.

In vorteilhafter Weise ist der Anschlag justierbar ausgebildet. Dadurch lässt sich die ßefestigungsvorrichtung an die Beschaffenheit des Knochens anpassen. Zum einen lässt sich eine vom Knochen abhängige Einschraubtiefe der Schraube ausgleichen, zum anderen kann die Vorspannung der Feder an die Härte des Knochens angepasst werden. Der Anschlag besteht beispielsweise aus einer Überwurfmutter am Anschlagelement oder an der Schraube. Die Überwurfmutter ist in Richtung der Feder, also entlang der Schraubenachse, verstellbar. Die Überwurfmutter ist bevorzugt gegenüber dem Anschlagelement beziehungsweise der Schraube fixierbar, zum Beispiel mittels einer Klemmschraube.

Bei der Feder handelt es sich beispielsweise um eine Schraubenfeder. In einer Ausgestaltungsform ist die Schraubenfeder auf die Schraube aufgeschoben, sodass die Schraube in Achsrichtung innerhalb der Schraubenfeder verläuft. Ein Ende der Schraubenfeder liegt am Fixierelement an, das andere Ende an einem Anschlag an der Schraube. Die Schraube und die Schraubenfeder beziehungsweise das Fixierelement sind insbesondere konzentrisch angeordnet.

In einer anderen Ausgestaltungsform ist die Schraubenfeder auf das Anschlagelement aufgeschoben, wobei sich das Anschlagelement bevorzugt durch die Feder in das Fixierelement hinein erstreckt. Die Schraube verläuft, bevorzugt konzentrisch, durch das Anschlagelement, die Schraubenfeder und das Fixierelement. In dieser Ausgestaltungsform stellen das Fixierelement, das Anschlagelement und die Feder eine einfach handhabbare Einheit dar, die in einem einzigen Arbeitsschritt am Knochen fixierbar ist.

Dabei ist das Fixierelement beispielsweise hülsenartig ausgebildet. Das Fixierelement weist also einen hohlzylinderförmigen Grundteil auf, in dessen Innenbereich sich die Schraube erstreckt. Die Achse des Hohlzylinders ist dabei im Wesentlichen identisch mit oder parallel zu der Achse der Schraube. Die Halterung für die Markervorrichtung befindet sich bevorzugt an dem der Feder zugewandten, also dem dem Knochen abgewandten, Ende des Fixierelements. Durch die Ausdehnung des Fixierelements in Achsrichtung ergibt sich somit ein Abstand der Markervorrichtung vom Knochen, der Raum für das den Knochen umgebende Gewebe lässt.

Alternativ ist die Fixiereinrichtung flächig ausgebildet. Das bedeutet, dass die Ausdehnung des Fixierelements in Achsrichtung der Schraube geringer ist als in einer dazu senkrechten Richtung, beispielsweise maximal 50%, 25%, 10% oder 5% dieser Ausdehnung beträgt. Die Halterung für die Markervorrichtung besteht dann vorteilhaft aus einem Ausleger am Fixierelement.

Alternativ handelt es sich bei der Feder um eine Tellerfeder oder Blattfeder. Eine solche Feder eignet sich insbesondere, wenn das Fixierelement eine flache, beispielsweise tellcrartige, Grundform aufweist. Eine Befestigungsvorrichtung mit einer Kopfschraube, einem flachen Fixierelement und einer Tellerfeder oder Blattfeder eignet sich insbesondere zur Anordnung zwischen dem Knochen und dem den Knochen umgebenden Gewebe. Die Halterung für die Markervorrichtung ist in diesem Fall beispielsweise ein Ausleger, der sich vom flachen Grundteil des Fixierelements durch das den Knochen umgebende Gewebe hindurch erstreckt.

In einer Ausgestaltungsform der Erfindung weist die Vorrichtung eine Schneideeinrichtung am Fixierelement auf. Die Schneideeinrichtung dient dazu, die Inzision, durch die die Befestigungsvorrichtung in den Körper eintritt, bedarfsgerecht zu vergrößern. So wird die Inzision z. B. zunächst nur so groß ausgelegt, dass die Befestigungsvorrichtung durch sie im Knochen verankert werden kann. Bewegt sich das Gewebe später in Bezug auf den Knochen, so wird die Inzision durch die Schneideeinrichtung gerade so weit vergrößert, dass die Befestigungsvorrichtung aus dem Körper austreten kann. Somit werden insbesondere von dem Gewebe auf die Befestigungsvorrichtung ausgeübte Momente minimiert.

Bevorzugt ist die Schneideeinrichtung gegenüber dem Fixierelement verdrehbar angeordnet. Die Achse, um die die Verdrehung der Schneideeinrichtung gegenüber dem Fixierelement möglich ist, entspricht dabei insbesondere der Achse, in der sich das Fixierelement gegenüber der Schraube verdrehen kann. Durch die verdrehbare Anordnung kann sich die Schneideeinrichtung automatisch in die Schnittrichtung ausrichten.

Bei der Schneideeinrichtung handelt es sich insbesondere um ein oder mehrere Messer. Die Schneiden der Messer erstrecken sich beispielsweise in Achsrichtung der Schraube.

Die vorliegende Erfindung soll anhand von zwei Ausführungsbeispielen näher erläutert werden. Dabei zeigt
- Figur 1: eine erste Bauform der Befestigungsvorrichtung,
- Figur 2: einen justierbaren Federanschlag,
- Figur 3: eine Stützplatte,
- Figur 4: die Befestigungsvorrichtung aus Figur 1 mit weiteren Ausstattungsmerkmaten,
- Figur 5: eine zweite Bauform der Befestigungsvorrichtung vor der Anbringung, und
- Figur 6: die Befestigungsvorrichtung aus Figur 5 im verschraubten Zustand.

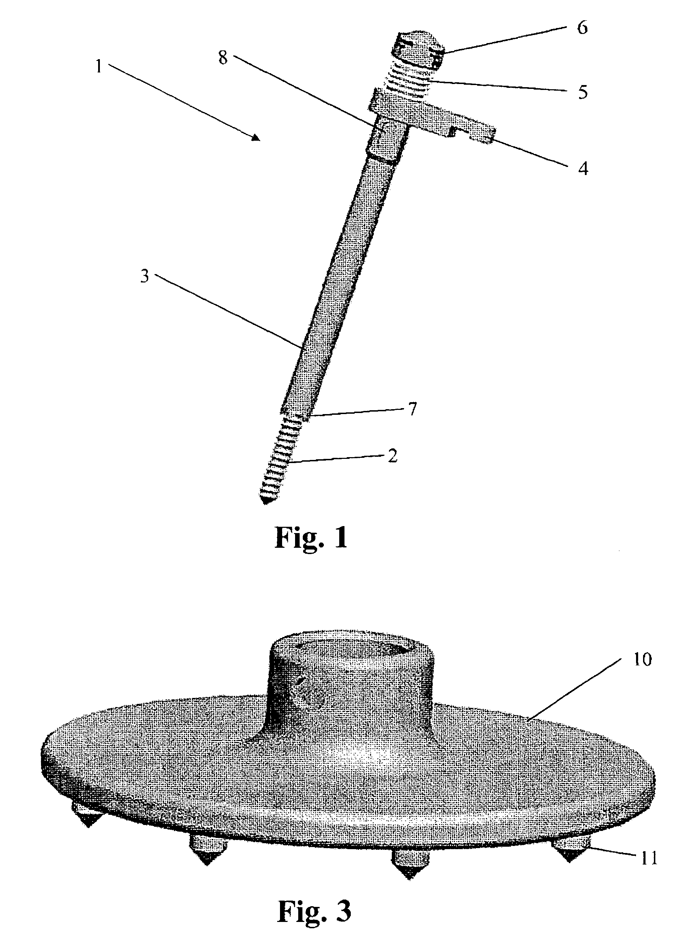

Die Figur 1 zeigt eine Vorrichtung 1 zur Befestigung einer Markervorrichtung in einem Knochen gemäß einer ersten Bauform. Aus Gründen der Übersichtlichkeit wurden in sämtlichen Figuren sowohl die Markervorrichtung als auch der Knochen weggelassen.

Die Vorrichtung 1 weist ein Fixierelement 3 und eine Halterung 4 für die Markervorrichtung am Fixierelement 3 auf. Das Fixierelement 3 ist hülsenartig ausgestaltet, weist also einen hohlzylindrischen Grundkörper auf. An einer Stirnfläche des hohlzylindrischen Grundkörpers weist das Fixierelement 3 Spikes 7 auf, die bei der Anbringung der Vorrichtung 1 in den Knochen eindringen und eine Verdrehung des Fixierelements 3 gegenüber dem Knochen verhindern. An der gegenüberliegenden Stirnfläche des Fixierelements 3 liegt eine Feder 5 an, die außerdem an einem Anschlag anliegt. Der Anschlag wird von einer Fläche eines Anschlagelements 6 gebildet, das teleskopartig gegenüber dem Fixierelement 3 in Achsrichtung des hohlzylinderförmigen Grundkörpers verschiebbar ist und mit einem Ende durch die Feder 5 hindurch in das Fixierelement 3 eintaucht.

Bei der Anbringung der Vorrichtung 1 wird das Fixierelement 3 in der gewünschten Ausrichtung auf den Knochen aufgesetzt, die Schraube 2 durch die Vorrichtung 1 hindurchgesteckt und in den Knochen eingeschraubt. Dabei verläuft die Schraube 2 durch das Fixierelement 3, die Feder 5 und das Anschlagelement 6, beispielsweise konzentrisch. Ab einer gewissen Einschraubtiefe der Schraube 2 in den Knochen drückt der Anschlag gegen die Feder 5 und komprimiert diese. Dies erzeugt eine Federkraft, die bewirkt, dass die Spikes 7 in den Knochen eindringen und somit eine Verdrehung des Fixierelements 3 gegenüber dem Knochen bzw. der Schraube 2 verhindern.

Das Fixierelement 3 ist in Richtung der Schraubenachse gegenüber der Schraube 2 verschiebbar. Außerdem ist das Fixierelement 3 um die Schraube 2 drehbar, wobei die Drehachse im Wesentlichen der Schraubenachse der Schraube 2 entspricht. Dazu weist der hohlzylinderförmige Grundkörper des Fixierelements 3 einen Innendurchmesser auf, der geringfügig größer ist als der Außendurchmesser des Gewindes der Schraube 2. Optional ist der Teil der Schraube 2, der durch das Fixierelement 3 verläuft, gewindefrei.

Das Anschlagelement 6 liegt am Kopf der Schraube 2 an, um die von der Feder aufgebrachte Kraft auf die Schraube 2 zu übertragen. Alternativ dient der Kopf der Schraube 2 ummittelbar als Anschlag für die Feder 5, in diesem Fall ist also kein Anschlagelement 6 vorhanden. Eine Reinigungsöffnung 8 an dem der Feder zugewandten Ende des Fixierelements 3 dient der Reinigung der Vorrichtung 1.

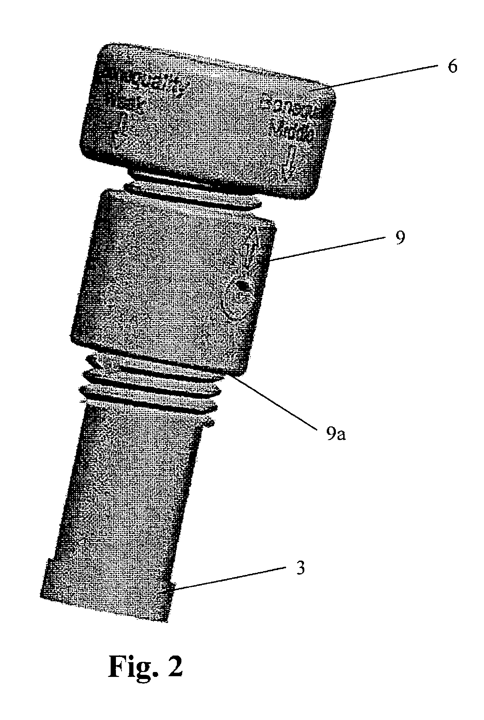

Die Figur 2 zeigt einen optionalen Mechanismus für einen justierbaren Anschlag. In diesem Fall dient nicht der Kopf des Anschlagelements 6 direkt als Anschlag für die Feder 5, sondern die Fläche 9a einer Überwurfmutter 9, die auf das Anschlagelement 6 aufgeschraubt ist. Durch eine Drehung der Überwurfmutter 9 verschiebt sich diese in Achsrichtung der Schraube 2 gegenüber dem Anschlagelement 6 und somit gegenüber dem Kopf der Schraube 2. Dies hat zur Folge, dass sich die Eindringtiefe der Schraube 2 in den Knochen ändert, ab der sich die Feder 5 vorzuspannen beginnt. Dadurch lässt sich die Vorrichtung 1 an die Eigenschaften des Knochens anpassen, indem die Eindringtiefe der Schraube 2 in den Knochen justiert wird, bei der die Schraube 5 die gewünschte Vorspannung aufweist. Weiterhin ergibt sich eine Anpassung der Federkraft an die Härte des Knochens. Alternativ befindet sich die Überwurfmutter 9 direkt auf der Schraube 2, wenn kein separates Anschlagelement 6 vorhanden ist.

Dargestellt in der Figur 3 ist eine Stützplatte 10, die auf das Fixierelement 3 aufgeschoben und mit diesem verschraubt wird. Die Stützplatte 10 dient der großflächigen Abstützung der Vorrichtung 1 auf dem Knochen, damit auf die Vorrichtung 1 wirkende Momente kein Verkippen der Vorrichtung gegenüber dem Knochen verursachen. Dazu wird die Stützplatte 10 genau so weit auf das Fixierelement 3 aufgeschoben, dass die Spikes 7 auf der dem Knochen zugewandten Seite des Fixierelements 3 noch über die Stützplatte 10 hinausragen. Die Stützplatte 10 weist zusätzliche Spikes 1 auf, die wie die Spikes 7 eine Verdrehung des Fixierelements 3 gegenüber dem Knochen verhindern. Es ist optional möglich, die Stützplatte 10 einstückig mit dem Fixierelement 3 auszubilden.

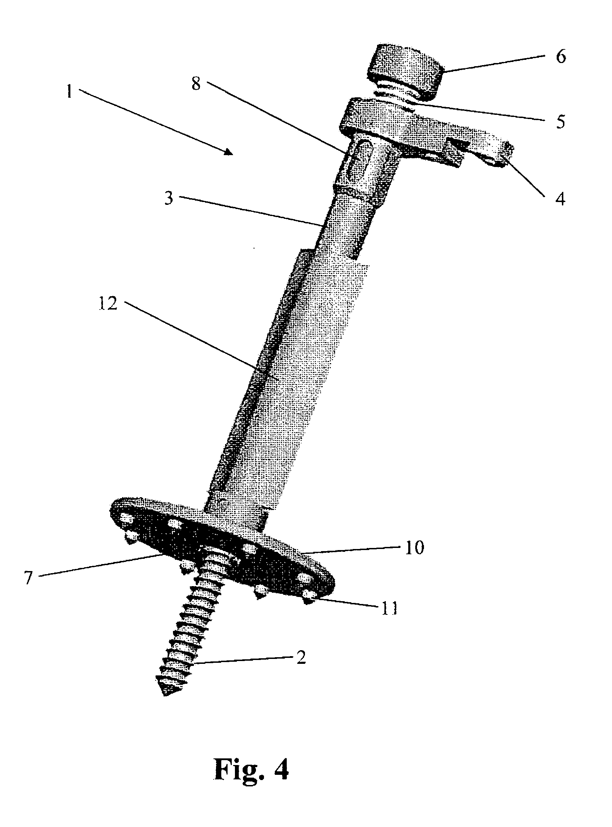

Die Figur 4 zeigt die Vorrichtung 1 mit der zusätzlichen Stützplatte 10 und einer Schneideeinrichtung 12. Die Schneideeinrichtung 12 umgibt das Fixierelement und weist zwei Klingen auf, die sich in Achsrichtung des hohlzylinderförmigen Grundteils des Fixierelements 3, also der Schraube 2, erstrecken. Die beiden Schneiden befinden sich an diametral gegenüberliegenden Seiten der Schneideeinrichtung 12. Die Schneideeinrichtung 12 ist um die Längsachse des Fixierelements 3 gegenüber dem Fixierelement 3 verdrehbar. Sie dient dazu, die Inzision, durch die die Vorrichtung 1 auf den Knochen aufgesetzt und mit diesem verschraubt wird, bei Bedarf zu erweitern. Dabei wird das den Knochen umgebende Gewebe gerade so weit aufgeschnitten, dass keine oder nur noch eine geringe Kraft auf die Vorrichtung 1 ausgeübt wird.

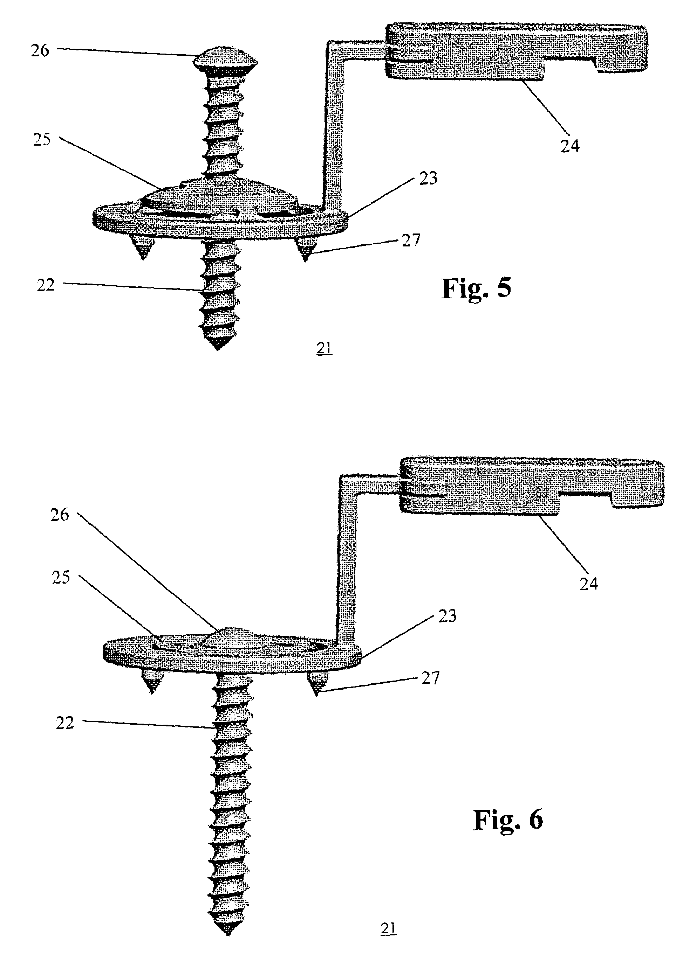

Die Figuren 5 und 6 zeigen eine Vorrichtung 21 zur Befestigung einer Markervorrichtung an einem Knochen gemäß einer zweiten Bauform mit einem Fixierelement 23 und einer Tellerfeder 25. Dabei ist das Fixierelement 23 im Gegensatz zum Fixierelement 3 flächig anstatt hülsenartig ausgebildet. Von dem flächigen, im vorliegenden Ausführungsbeispiel kreisscheibenförmigen Grundkörper des Fixierelements 23 erstreckt sich ein Ausleger als Halterung 24 für die Markervorrichtung. Eine Schraube 22 dient der Fixierung der Vorrichtung 21 im Knochen.

Die Schraube 22 durchdringt die Tellerfeder 25 und das kreisscheibenförmige Grundteil des Fixierelements 23 jeweils in deren Mittelpunkten. Die Tellerfeder 25 ist zwischen dem Fixierelement 23 und dem Kopf 26 der Schraube 22 angeordnet. Der Kopf 26 der Schraube 22 dient als Anschlag für die Tellerfeder 25. Auf der der Tellerfeder 25 gegenüberliegenden Seite des Fixierelements 23 sind Spikes 27 angeordnet, die in den Knochen eindringen und eine Verdrehung des Fixierelements 23 gegenüber dem Knochen verhindern. Das Fixierelement 23 ist um eine Schraube 22 drehbar und in Richtung der Schraubenachse gegenüber der Schraube 22 verschiebbar.

Die Figur 5 zeigt die Vorrichtung 21 vor der Anbringung am Knochen. Bei der Anbringung wird das Fixierelement 23 auf den Knochen aufgelegt und gegenüber dem Knochen ausgerichtet. Anschließend wird die Schraube 22 durch die Vorrichtung 21 in den Knochen hineingedreht. Ab einer gewissen Eindringtiefe der Schraube 22 in den Knochen liegt der Kopf 26 der Schraube 22 an der Tellerfeder 25 an und komprimiert diese. Ab diesem Zeitpunkt erzeugt die Tellerfeder 25 eine Kraft auf das Fixierelement 23, die die Spikes 27 in den Knochen drückt. Beim weiteren Hineindrehen der Schraube 22 in den Knochen wird die Tellerfeder 25 zunehmend vorgespannt.

Der Endzustand bei vollständig in den Knochen eingeschraubter Schraube 22 ist in Figur 6 dargestellt. Wird der Knochen aufgrund der Krafteinwirkung durch die Spikes 27 weich, so entspannt sich die Tellerfeder 25 und drückt die Spikes 27 tiefer in den Knochen hinein. Dadurch wird die Fixierung des Fixierelements 23 gegenüber dem Knochen sichergestellt, ohne dass die Schraube 22 nachgezogen werden müsste. Optional ist mehr als eine Schraube zur Fixierung der Vorrichtung im Knochen vorgesehen, wobei bevorzugt für jede Schraube eine Feder vorhanden ist.

Es ist möglich, einzelne Merkmale der Ausführungsbeispiele miteinander zu kombinieren. So ist es beispielsweise möglich, die Vorrichtung 21 mit einem justierbaren Anschlag zu versehen oder bei der Vorrichtung 1 eine Tellerfeder anstatt einer Schraubenfeder zu verwenden.

## Patentansprüche

1. Vorrichtung (1, 21) zur Befestigung einer Markervorrichtung an einem Knochen, aufweisend eine in dem Knochen fixierbare Schraube (2, 22), einen Anschlag, der Teil der Schraube (2, 22) ist oder von einem Anschlagelement gebildet wird, das von der Schraube (2, 22) abgestützt wird, ein Fixierelement (3, 23), durch das die Schraube hindurchführbar ist, mit einer Halterung (4, 24) für die Markervorrichtung sowie eine Feder (5, 25), **dadurch gekennzeichnet, dass** die Feder (5, 25) derart zwischen dem Anschlag und dem Fixierelement (3, 23) angeordnet ist, dass durch sie das Fixierelement (3, 23) relativ zur Schraube (2, 22) gegen den Knochen drückbar ist.

2. Vorrichtung (1, 21) nach Anspruch1, **dadurch gekennzeichnet, dass** die dem Knochen zugewandte Seite des Fixierelementes (3) eine Stützplatte (10) aufweist.

3. Vorrichtung (1, 21) nach einem der Ansprüche 1 oder 2, **gekennzeichnet durch** mindestens einen Spike (7) an der dem Knochen zugewandten Seite des Fixierelements (3, 23).

4. Vorrichtung (1, 21) nach Anspruch 1, **dadurch gekennzeichnet, dass** der Anschlag am Anschlagelement justierbar ausgebildet ist.

5. Vorrichtung (1, 21) nach Anspruch 4, **dadurch gekennzeichnet, dass** der Anschlag aus einer Überwurfmutter (9) besteht, die in Richtung der Feder (5, 25) verstellbar ist.

6. Vorrichtung (1, 21) nach einem der Ansprüche 1 bis 5, **dadurch gekennzeichnet, dass** es sich bei der Feder (25) um eine Schraubenfeder, eine Tellerfeder oder eine Blattfeder handelt.

7. Vorrichtung (1, 21) nach einem der Ansprüche 1 bis 6, **gekennzeichnet durch** eine Schneideeinrichtung (12) am Fixierelement (3).

8. Vorrichtung (1, 21) nach Anspruch 7, **dadurch gekennzeichnet, dass** die Schneideeinrichtung (12) gegenüber dem Fixierelement (3) verdrehbar angeordnet ist.

9. Vorrichtung (1, 21) nach einem der Ansprüche 7 oder 8, **dadurch gekennzeichnet, dass** es sich bei der Schneideeinrichtung (12) um ein Messer handelt.

10. Vorrichtung (1, 21) nach einem der Ansprüche 1 bis 9, wobei sich die Schraube (2, 22) durch das Fixierelement (3, 23) und die Feder (5, 25) erstreckt.

11. Vorrichtung nach einem der Ansprüche 1 bis 10, **dadurch gekennzeichnet, dass** das Fixierelement (3, 23) gegenüber der Schraube (2, 22) in Richtung der Schraubenachse verschiebbar angeordnet ist.

12. Vorrichtung nach einem der Ansprüche 1 bis 11, **dadurch gekennzeichnet, dass** das Fixierelement (3, 23) gegenüber der Schraube (2, 22) um die Schraubenachse verdrehbar angeordnet ist.

## Claims

1. A device (1, 21) for fastening a marker device to a bone, comprising a screw (2, 22) which can be fixed in the bone, an abutment which is part of the screw (2, 22) or is formed by an abutment element which is supported by the screw (2, 22), a fixing element (3, 23) through which the screw (2, 22) can be guided and which comprises a mounting (4, 24) for the marker device, and a spring (5, 25), **characterised in that** the spring (5, 25) is arranged between the abutment and the fixing element (3, 23) such that the fixing element (3, 23) can be pressed against the bone relative to the screw (2, 22) by the spring (5, 25).

2. The device (1, 21) according to claim 1, **characterised in that** the side of the fixing element (3) facing the bone comprises a supporting plate (10).

3. The device (1, 21) according to any one of claims 1 or 2, **characterised by** at least one spike (7) on the side of the fixing element (3, 23) facing the bone.

4. The device (1, 21) according to claim 1, **characterised in that** the abutment on the abutment element is designed to be adjustable.

5. The device (1, 21) according to claim 5, **characterised in that** the abutment consists of a screw nut (9) which can be adjusted in the direction of the spring (5, 25).

6. The device (1, 21) according to any one of claims 1 to 5, **characterised in that** the spring (25) is a helical spring, a disc spring or a leaf spring.

7. The device (1, 21) according to any one of claims 1 to 6, **characterised by** a cutting device (12) on the fixing element (3).

8. The device (1, 21) according to claim 7, **characterised in that** the cutting device (12) is arranged such that it can be rotated relative to the fixing element (3).

9. The device (1, 21) according to any one of claims 7 or 8, **characterised in that** the cutting device (12) is a knife.

10. The device (1, 21) according to any one of claims 1 to 9, wherein the screw (2, 22) extends through the fixing element (3, 23) and the spring (5, 25).

11. The device according to any one of claims 1 to 10, **characterised in that** the fixing element (3, 23) is arranged such that it can be shifted relative to the screw (2, 22) in the direction of the screw axis.

12. The device according to any one of claims 1 to 11, **characterised in that** the fixing element (3, 23) is arranged such that it can be rotated relative to the screw (2, 22) about the screw axis.

## Revendications

1. Dispositif (1, 21) destiné à fixer un dispositif marqueur sur un os, comportant une vis (2, 22) pouvant être fixée dans l'os, une butée faisant partie de la vis (2, 22) ou formée par un élément de butée qui est supporté par la vis (2, 22), un élément de fixation (3, 23) à travers lequel la vis peut passer, avec un support (4, 24) pour le dispositif marqueur ainsi qu'un ressort (5, 25), **caractérisé en ce que** le ressort (5, 25) est agencé entre la butée et l'élément de fixation (3, 23) de telle sorte qu'il permet de pousser l'élément de fixation (3, 23) par rapport à la vis (2, 22) contre l'os.

2. Dispositif (1, 21) selon la revendication 1, **caractérisé en ce que** le côté de l'élément de fixation (3) dirigé vers l'os comporte une plaque de support (10).

3. Dispositif (1, 21) selon l'une quelconque des revendications 1 ou 2, **caractérisé par** au moins une pointe (7) sur le côté de l'élément de fixation (3, 23) dirigé vers l'os.

4. Dispositif (1, 21) selon la revendication 1, **caractérisé en ce que** la butée est formée sur l'élément de butée de manière à pouvoir être réglée.

5. Dispositif (1, 21) selon la revendication 4, **caractérisé en ce que** la butée est constituée d'un écrou de raccord (9) qui peut être réglé dans la direction du ressort (5, 25).

6. Dispositif (1, 21) selon l'une quelconque des revendications 1 à 5, **caractérisé en ce que** le ressort (25) est un ressort hélicoïdal, un ressort Belleville ou un ressort à lames.

7. Dispositif (1, 21) selon l'une quelconque des revendications 1 à 6, **caractérisé par** un dispositif de coupe (12) sur l'élément de fixation (3).

8. Dispositif (1, 21) selon la revendication 7, **caractérisé en ce que** le dispositif de coupe (12) est agencé de manière rotative par rapport à l'élément de fixation (3).

9. Dispositif (1, 21) selon l'une quelconque des revendications 7 ou 8, **caractérisé en ce que** le dispositif de coupe (12) est un couteau.

10. Dispositif (1, 21) selon l'une quelconque des revendications 1 à 9, dans lequel la vis (2, 22) s'étend à travers l'élément de fixation (3, 23) et le ressort (5, 25).

11. Dispositif selon l'une quelconque des revendications 1 à 10, **caractérisé en ce que** l'élément de fixation (3, 23) est agencé de manière mobile par rapport à la vis (2, 22) dans la direction de l'axe de vis.

12. Dispositif selon l'une quelconque des revendications 1 à 11, **caractérisé en ce que** l'élément de fixation (3, 23) est agencé de manière rotative autour de l'axe de vis par rapport à la vis (2, 22).
